# FASCICULE DE BREVET EUROPEEN

(11) **EP 1 424 059 B1**
(45) Date de publication et mention de la délivrance du brevet: **07.06.2006**
(21) Numéro de dépôt: 03292110.8
(22) Date de dépôt: 27.08.2003
(51) Int. Cl.: A61Q 1/00, A61K 8/92

(54) **Composition cosmétique comprenant une cire collante**
Kosmetische Zusammensetzung die ein klebriges Wachs enthält
Cosmetic composition comprising a tacky wax

(30) Priorité: 06.09.2002 FR 0211096; 06.09.2002 FR 0211104; 06.09.2002 FR 0211097; 30.09.2002 FR 0212097; 30.09.2002 FR 0212098
(43) Date de publication de la demande: 02.06.2004
(73) Titulaire: L'OREAL, 75008 Paris (FR)
(72) Inventeur: de la Poterie, Valérie, 77820 Le Chatelet en Brie (FR); Daubige, Thérèse, 77480 Mousseaux les Bray (FR); Styczen, Patrice, 91190 Gif-sur-Yvette (FR)
(74) Mandataire: Kromer, Christophe

(56) Documents cités:
- EP-A- 0 987 002
- EP-A- 1 201 222
- DE-A- 19 751 221
- US-A- 5 783 176
- DONATAS SATAS: "Handbook of Pressure Sensitive Adhesive Technology" 1999, SATAS & ASSOCIATES , WARWICK, US * page 36 - page 47 *

## Description

La présente invention a pour objet une composition cosmétique comprenant une cire collante. Cette composition est utilisable notamment dans le domaine du maquillage ou du soin des matières kératiniques comme la peau, les ongles, les cils, les sourcils, les cheveux, d'être humain. L'invention concerne aussi un procédé cosmétique de soin ou de maquillage des matières kératiniques.
La composition peut se présenter sous forme d'un produit de revêtement des fibres kératiniques telles que les sourcils, les cheveux, les cils ou , sous forme d'eye-liner, de fard à paupières, de fard à joue, de fond de teint, de produit pour les lèvres, de produit de maquillage du corps (tatouage semi-permanent), . Plus spécialement, l'invention porte sur une composition de soin ou de maquillage des fibres kératiniques, et notamment sur une composition de soin ou de maquillage des cils ou mascara.

La composition de revêtement des fibres kératiniques peut être une composition de maquillage, une composition à appliquer sur un maquillage, dite encore top-coat, ou bien encore une composition de traitement (ou de soin) des cils, des sourcils ou des cheveux dite encore basecoat.

Les mascaras sont couramment préparés selon deux types de formulation : les mascaras aqueux, dits mascaras crèmes, sous forme d'émulsion de cires dans l'eau ; les mascaras anhydres ou à faible teneur en eau, dits mascaras waterproofs, sous forme de dispersions de cires dans des solvants organiques.
Il est connu d'employer diverses cires pour la formulation des mascaras comme celles décrites dans le document WO-A-91/12793, par exemple la cire d'abeille, la cire de candellila,la cire de carnauba, ou bien encore la cire de polyéthylène.

Toutefois, lorsque les mascaras contiennent certaines cires en quantité importante (au dessus de 20 % en poids du poids total du mascara) comme la cire de carnauba, la cire de son de riz ou la cire de polyéthylène, le dépôt du maquillage des cils obtenu présente un aspect granuleux conférant ainsi un maquillage non lisse et non homogène, défauts qui rendent le maquillage inesthétique.

Par ailleurs, pour obtenir un mascara présentant de bonnes propriétés chargeantes, c'est-à-dire obtenir un maquillage épais des cils, il est possible d'incorporer dans le mascara une ou plusieurs cires en une teneur totale supérieure à 25 % en poids du poids total du mascara. Or en utilisant les cires classiques comme la cire d'abeille, la cire de candellila, ou la cire de carnauba dans ces teneurs élevées, la composition de mascara devient très consistante, voire trop compacte, et ne peut pas être appliquée facilement sur les cils avec les applicateurs de brosse à mascara couramment utilisés. Le mascara trop épais est déposé sur les cils sous forme de paquets et le maquillage ainsi obtenu ne présente pas l'aspect lisse recherché ; le maquillage n'est pas homogène et a un aspect inesthétique.
En outre, certaines cires comme la cire d'orange, la cire de lanoline, employées à des teneurs supérieures à 25 % en poids conduisent à des compositions qui ne sont pas suffisamment stables, notamment après un stockage de deux semaines à température ambiante (25 °C) : la composition prend en masse (augmentation importante de la viscosité) ou présente un déphasage qui s'observe visuellement à l'oeil nu. La composition est alors inappropriée pour être appliquée sur les matières kératiniques.

Le but de la présente invention est de proposer une composition de maquillage ou de soin des matières kératiniques comprenant un taux élevé de cire permettant d'obtenir un dépôt homogène et lisse sur les matières kératiniques.

Un autre but de la présente invention est de disposer d'une composition cosmétique qui s'applique facilement sur les matières kératiniques et permettant un maquillage rapide desdites matières kératiniques, pouvant comprendre un taux élevé de cire.

Un autre but de l'invention est d'obtenir une composition cosmétique comprenant un taux élevé de cire qui soit stable, notamment après un stockage de 24 heures à 25 °C, voire pendant 15 jours.

Les inventeurs ont découvert qu'une telle composition pouvait être obtenue en utilisant une cire particulière présentant des propriétés collantes (collant élevé). Cette cire permet d'obtenir une composition cosmétique qui s'applique facilement sur les matières kératiniques, ayant une bonne accroche sur les matières kératiniques et qui permet une application rapide du maquillage et conduit à la formation d'un maquillage lisse et homogène, ne présentant pas d'aspect granuleux.
De plus, la cire collante peut être incorporée dans la composition en une teneur pouvant aller jusqu'à 60 % en poids, par rapport au poids total de la composition, sans obtenir une prise en masse de la composition : la composition est stable (notamment stabilité après 24 heures à 25 °C), reste d'une consistance crémeuse et s'applique facilement sur les matières kératiniques.

De façon plus précise, l'invention a pour objet une composition de maquillage ou de soin des matières kératiniques comprenant, dans un milieu cosmétiquement acceptable, au moins 27 % en poids, par rapport au poids total de la composition d'une première cire ayant un collant supérieur ou égal à 0,7 N.s et une dureté inférieure ou égale à 3,5 MPa.

L'invention a également pour objet un procédé cosmétique de maquillage ou de soin non thérapeutique des matières kératiniques, en particulier de la peau, des ongles, des cheveux, des cils, des sourcils, comprenant l'application sur les matières kératiniques d'une composition telle que définie précédemment.

L'invention a aussi pour objet l'utilisation d'une composition telle que définie précédemment pour obtenir un maquillage des matières kératiniques homogène et/ou lisse et/ou un maquillage rapide des matières kératiniques.

L'invention a encore pour objet l'utilisation d'une cire ayant un collant supérieur ou égal à 0,7 N.s et une dureté inférieure ou égale à 3,5 MPa, dans une composition cosmétique, pour obtenir un maquillage des matières kératiniques rapide et/ou homogène et/ou lisse et/ou obtenir une composition cosmétique stable, la cire étant présente en une teneur d'au moins 27 % en poids par rapport au poids total de la composition.

Pour certaines applications cosmétiques, il serait avantageux de pouvoir modérer le collant naturel de la cire collante tout en conservant les propriétés avantageuses de dépot lisse et homogène . Ainsi, dans le cas particulier des formules de type mascara, certaines utilisatrices cherchent à obtenir une parfaite individualisation des cils qui n'est pas toujours optimale en présence d'une cire collante.
D'une manière inattendue, les inventeurs ont constaté qu'il était possible de satisfaire à cette exigence supplémentaire sous réserve d'associer à ladite cire collante au moins un composé choisi parmi un ester de dextrine et d'acide(s) gras et/ou une charge possédant une surface spécifique BET supérieure ou égale à 100 m²/g.

C'est pourquoi l'invention a aussi pour objet l'utilisation d'au moins un composé choisi parmi un ester de dextrine et d'acide(s) gras et/ou une charge possédant une surface spécifique BET supérieure ou égale à 100 m²/g dans une composition cosmétique contenant au moins 27 % en poids, par rapport au poids total de la composition, d'au moins une cire ayant un collant supérieur ou égal à 0,7 N.s et une dureté inférieure ou égale à 3,5 MPa, pour obtenir un maquillage des matières kératiniques homogène et/ou lisse et notamment un maquillage des fibres kératiniques, chargeant et séparant et/ou obtenir une composition cosmétique stable.

On entend par « milieu cosmétiquement acceptable » un milieu cosmétique compatible avec les matières kératiniques comme, la peau y compris les lèvres, les ongles, les cheveux, les cils, les sourcils.

### Cire collante

La première cire, appelée aussi cire collante, présente dans la composition selon l'invention a un collant supérieur ou égal à 0,7 N.s, notamment allant de 0,7 N.s à 30 N.s, de préférence supérieur ou égal à 1 N.s , notamment allant de 1 N.s à 20 N.s, et préférentiellement supérieur ou égal à 2 N.s, notamment allant de 2 N.s à 10 N.s, et encore mieux allant de 2 N.s à 5 N.s.

La cire collante a une dureté inférieure ou égale à 3,5 MPa, en particulier allant de 0,01 à 3,5 MPa, de préférence allant de 0,05 MPa à 3 MPa, et plus préférentiellement allant de 0,1 MPa à 2,5 MPa.

Par "cire", on entend au sens de la présente invention, un composé gras lipophile, solide à température ambiante (25°C) et pression atmosphérique (760 mm de Hg, soit 105 Pa), à changement d'état solide/liquide réversible, ayant une température de fusion supérieure à 30°C et mieux supérieure à 55 °C et pouvant aller jusqu'à 200° C, notamment jusqu'à 120 °C.
En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10 °C par minute.

Le collant de la première cire est mesuré à 20 °C à l'aide du texturomètre vendu sous la dénomination TA-TX2i par la société RHEO, équipé d'un mobile en polymère acrylique en forme de cône formant un angle de 45°, en mesurant l'évolution de la force (force de compression ou force d'étirement) (F) en fonction du temps, pendant l'opération suivante :
Le mobile est déplacé à la vitesse de 0,5 mm /s puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s. Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

Pour effectuer la mesure du collant de la cire, la cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

La dureté de la première cire est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression maximale mesurée divisée par la surface du cylindre du tèxturomètre en contact avec la cire.

Comme cire collante, on peut utiliser un (hydroxystéaroyloxy)stéarate d'alkyle en C₂₀-C₄₀ (le groupe alkyle comprenant de 20 à 40 atomes de carbone) , en particulier un 12-(12'hydroxystéaroyloxy)stéarate d'alkyle en C₂₀-C₄₀, de formule (I) : 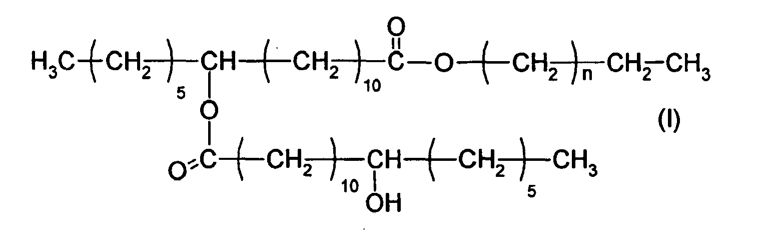 dans laquelle n est un entier allant de 18 à 38, ou un mélange de composés de formule (I).

Aussi, l'invention a également pour objet une composition de maquillage de soin des matières kératiniques comprenant, dans un milieu cosmétiquement acceptable, un (hydroxystéaroyloxy)stéarate d'alkyle en C₂₀-C₄₀ (en particulier un 12-(12'hydroxystéaroyloxy)stéarate d'alkyle en C₂₀-C₄₀), notamment de formule (I) comme décrit précédemment.

Une telle première cire est notamment vendue sous les dénominations « KESTER WAX K 82 P » et « KESTER WAX K 80 P » par la société KOSTER KEUNEN.

La première cire peut éventuellement se présenter sous forme de microdispersion aqueuse de cire.

On entend par microdispersion aqueuse de cire, une dispersion aqueuse de particules de cire, dans laquelle la taille desdites particules de cire est inférieure ou égale à environ 1 µm.

Les microdispersions de cire sont des dispersions stables de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.
Les microdispersion de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 0,99 µm), de préférence inférieures à 0,5 µm (notamment allant de 0,06 µm à 0,5 µm).
Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

La première cire ou cire collante peut être présente dans la composition selon l'invention en une teneur allant de 0,5 % à 60 % en poids, notamment de 1 % à 50 % en poids, en particulier allant de 10 % à 40 % en poids par rapport au poids total de la composition.
En particulier la cire collante est présente dans la composition selon l'invention en une teneur supérieure à 27 % en poids par rapport au poids total de la composition, par exemple allant de 27 à 60 % en poids, , de préférence supérieure à 28% en poids, par exemple de 28 à 50 % en poids et plus préférentiellement supérieure à 30 % en poids, par exemple de 30 à 40% en poids.

### Deuxième cire

Avantageusement, la composition selon l'invention comprend une deuxième cire, appelée aussi cire dure, qui a une dureté supérieure ou égale à 6 MPa , notamment allant de 6 MPa à 30 MPa, et de préférence supérieure ou égale à 7 MPa, notamment allant de 7 MPa à 25 MPa et, mieux supérieure ou égale à 8 MPa , notamment de 8 à 25 MPa, encore mieux supérieure ou égale à 9 MPa, par exemple de 9 à 20 MPa

La dureté de la cire dure est mesurée selon le même protocole décrit précédemment pour la première cire.

Comme cire dure, on peut utiliser la cire de Carnauba, de Candellila, les cires de polyéthylène, l'huile de jojoba hydrogénée, la cire de sumac, la cérésine, le stéarate d'octacosanyle, le stéarate de tétracontanyle, la cire de Shellac, le fumarate de béhényle, le tétrastéarate de di-(triméthylol-1,1,1 propane) vendu sous la dénomination « HEST 2T-4S» par la société HETERENE, le tétrabéhénate de di-(triméthylol-1,1,1 propane) vendue sous la dénomination HEST 2T-4B par la société HETERENE, les ozokerites comme celle vendue sous la dénomination « OZOKERITE WAX SP 1020 P » par la société STRAHL & PITSCH la cire obtenue par hydrogénation d'huile d'olive estérifiée avec l'alcool stéarylique vendue sous la dénomination PHYTOWAX Olive 18 L 57 par la Société SOPHIM.

La deuxième cire peut se présenter sous la forme d'une microdispersion aqueuse de particules de cire comme décrit pour la première cire.

La deuxième cire dure peut être présente dans la composition selon l'invention en une teneur allant de 0,1 % à 30 % en poids, par rapport au poids total de la composition, de préférence allant de 1% à 20 % en poids, et plus préférentiellement allant de 2 % à 10 % en poids.

Comme exposé plus haut, lorsque le collant doit être ajusté, il est avantageux d'associer à la cire collante (première cire) un composé choisi parmi un ester de dextrine et d'acide(s) gras et/ou une charge possédant une surface spécifique BET supérieure ou égale à 100 m²/g.

### Charge à surface spécifique

La charge peut posséder une surface spécifique supérieure ou égale à 100 m²/g, notamment variant de 100 à 5000 m²/g, en particulier de 150 à 1000 m²/g, voire de 200 à 800 m2/g.

Par « charge », on désigne des particules de toutes formes, insolubles dans le milieu de la composition, quelle que soit la température à laquelle la composition est fabriquée.
Par « charge à surface spécifique », on entend une charge possédant une surface spécifique mesurée selon la méthode BET supérieure ou égale à 100 m²/g.

La « surface spécifique BET » est déterminée selon la méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale (donc micropores compris) de la charge.
La quantité en charge dans la composition selon l'invention est généralement ajustée de manière à contrôler le collant de la cire associée, à la valeur souhaitée. Ainsi, les charges conformes à l'invention sont particulièrement avantageuses pour la préparation de compositions cosmétiques comprenant au moins 10 %, notamment 20 %, en particulier au moins 25 %, et mieux 27% en poids d'au moins une cire collante.

A titre illustratif, cette charge peut être présente dans la composition selon l'invention en une quantité allant de 0,1 à 25 %, notamment de 0,5 à 20 % et en particulier de 1 à 15 % en poids par rapport au poids total de la composition.

Avantageusement, la cire collante et la charge à surface spécifique sont présentes en une teneur telle que le rapport pondéral de la cire collante par rapport à la charge à surface spécifique varie de 350 à 0,1, notamment de 100 à 0,5, en particulier de 50 à 0,8 et mieux de 30 à 1.

Les particules composant la charge à surface spécifique peuvent avoir une taille moyenne variant de 0,01 à 100 µm, notamment de 0,1 à 50 µm, et mieux de 1 à 20 µm. Par « taille moyenne », on désigne la dimension donnée par la distribution granulométrique statistique à la moitié de la population, dites D50.

La charge à surface spécifique conforme à l'invention peut être notamment choisie parmi les charges organiques, minérales et/ou leurs mélanges.
La charge organique peut être choisie parmi les cires polyoléfiniques comme les cires de polyéthylène et notamment celles commercialisées sous la dénomination « Performalen 2000® » par la société New Phase Technology ou parmi les charges polymériques telles que le polymethylmethacrylate (PMMA) comme le Jurymer MB1® vendu par Nihon Junyaku ou le polytétrafluoroéthylène (PTFE). A titre représentatif et non limitatif des charges minérales, on peut notamment citer les silices, les silicates, les alumines, les aluminosilicates, notamment celles commercialisées sous la dénomination « Sunsil 130® » par la Société Sunjin Chemical ou « Silicabeads SB150® »par la Société Miyoshi.

Les particules composant la charge à surface spécifique peuvent présenter des formes variées. Ces particules peuvent être notamment globulaires, lamellaires, en particulier sphériques, creuses ou pleines. Les charges creuses s'avèrent particulièrement avantageuses.
Ainsi, dans le cas particulier d'une charge minérale, conviennent tout particulièrement les microsphères de silice creuse, et en particulier telles que le « Sunsphère H-51» d'Asahi Glass de surface spécifique égale à 770 m²/g, et le « Sunsil 130» de Sunjin Chemical de surface spécifique 200-260 m²/g.

Selon un mode de réalisation particulier de l'invention, la composition comprend au moins une cire collante et au moins une charge possédant une surface spécifique supérieure ou égale à 100 m²/g, telle que de la silice creuse et sphérique, des microsphères de silice creuse, et/ou de la cire de polyéthylène.

### Ester de dextrine et d'acide(s) gras

L'ester de dextrine et d'acide(s) gras qui peut être associé à la cire collante dans la composition selon l'invention est plus particulièrement, un mono-ou poly-ester de dextrine et d'au moins un acide gras et notamment répondant à la formule (II) : 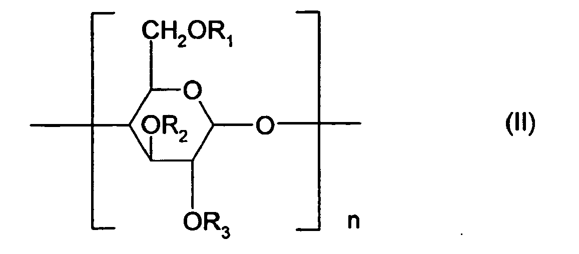 dans laquelle :
- n est un entier allant de 3 à 200, notamment allant de 20 à 150, et en particulier allant de 25 à 50,
- les radicaux R₁, R₂ et R₃, identiques ou différents, sont choisis parmi l'hydrogène ou un groupement acyle (R-CO-) dans lequel le radical R est un groupement hydrocarboné, linéaire ou ramifié, saturé ou insaturé, possédant de 6 à 50, mieux de 7 à 29, en particulier de 7 à 21, notamment de 11 à 19, plus particulièrement de 13 à 17, voire 15, atomes de carbone, sous réserve qu'au moins un desdits radicaux R₁, R₂ ou R₃ est différent de l'hydrogène.
   En particulier, R₁, R₂ et R₃ peuvent représenter l'hydrogène ou un groupement acyle (R-CO-) dans lequel R est un radical hydrocarboné tel que défini précédemment, sous réserve qu'au moins deux desdits radicaux R₁, R₂ ou R₃ sont identiques et différents de l'hydrogène.
   L'ensemble des radicaux R₁, R₂ et R₃ peuvent figurer un groupement acyle (R-CO) identique ou différent et notamment identique.
   En particulier, n varie avantageusement de 25 à 50, notamment est égal à 38 dans la formule générale (II) de l'ester selon l'invention.
   Notamment lorsque les radicaux R₁, R₂ et/ou R₃, identiques ou différents figurent un groupement acyle (R-CO), ceux-ci peuvent être choisis parmi les radicaux caprylyle, caproyle, lauroyle, myristyle, palmityle, stéaryle, eicosanyle, docosanoyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécénoyle, palmitoléyle, oléyle, élaidyle, eicosényle, sorbyle, linoléyle, linolényle, punicyle, arachidonyle, stéarolyle, et leurs mélanges.
   De préférence, on utilise à titre d'ester de dextrine et d'acide(s) gras au moins un palmitate de dextrine. Celui-ci peut être utilisé seul ou en mélange avec d'autres esters.

Avantageusement, l'ester de dextrine et d'acide gras a un degré de substitution inférieur ou égal à 2,5 sur la base d'une unité glucose, notamment variant de 1,5 à 2,5, de préférence de 2 à 2,5. Le poids moléculaire moyen en poids de l'ester de dextrine peut être en particulier de 10 000 à 150 000, notamment de 12 000 à 100 000 et voire de 15 000 à 80 000.

Des esters de dextrine, en particulier des palmitates de dextrine, sont disponibles commercialement sous la dénomination RHEOPEARL TL ou RHEOPEARL KL de la société Chiba Flour.

L'ester de dextrine peut être présent dans la composition selon l'invention à une quantité allant de 0,1 à 20 %, en particulier de 0,5 à 15 % en poids, et notamment de 1 à 10 % en poids, par rapport au poids total de la composition.

Avantageusement, la cire collante et l'ester de dextrine sont présents en une teneur telle que le rapport pondéral de la cire collante par rapport à l'ester de dextrine varie de 350 à 0,1, en particulier de 100 à 0,5, notamment de 50 à 1, voire de 15 à 2.

### Milieu physiologiquement acceptable

Le milieu physiologiquement acceptable de la composition peut comprendre un solvant volatil, notamment choisi parmi l'eau, les solvants organiques volatils et les huiles volatiles définis ci-après, et leurs mélanges.

La composition selon l'invention peut comprendre un milieu aqueux, constituant une phase aqueuse, qui peut former la phase continue de la composition.

La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄.
La phase aqueuse (eau et éventuellement le solvant organique miscible à l'eau) peut être présente, en une teneur allant de 1 % à 95 % en poids, par rapport au poids total de la composition, de préférence allant de 3 % à 80 % en poids, et préférentiellement allant de 5 % à 60 % en poids.

La composition selon l'invention peut comprendre une huile ou solvant organique qui peut notamment former une phase grasse, et en particulier une phase grasse continue. La composition peut être une composition anhydre.

Par " huile ou solvant organique volatile", on entend au sens de l'invention tout milieu non aqueux susceptible de s'évaporer au contact de la peau ou de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).. Par "huile non volatile", on entend une huile restant sur la peau ou la fibre kératinique à température ambiante et pression atmosphérique au moins plusieurs heures et ayant notamment une pression de vapeur inférieure à 10⁻³ mm de Hg (0,13Pa).

Ces huiles peuvent être des huiles hydrocarbonées, des huiles siliconées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C₈-C₁₆ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 6 centistokes (6 10⁻⁶ m²/s), et ayant notamment de 2 à 10 atomes de silicium, ces silicones comportant éventuellement des groupes alkyle ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention en une teneur allant de 0,1 % à 98 % en poids, par rapport au poids total de la composition, de préférence de 1% à 65 % en poids.

La composition peut également comprendre au moins une huile non volatile, et notamment choisie parmi les huiles hydrocarbonées et/ou siliconées non volatiles.
Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₅ + R₆ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles non volatiles peuvent être présentes dans la composition selon l'invention en une teneur allant de 0 à 50 % (notamment de 0,1 à 50 %) en poids, de préférence de 0 % à 40 % en poids (notamment 0,1 à 40 %), par rapport au poids total de la composition, et mieux de 0 % à 30 % en poids (notamment 0,1 % à 30 %).

La composition selon l'invention peut comprendre en outre une cire additionnelle, différente de la première cire collante et de la deuxième cire dure décrites précédemment. La cire additionnelle peut être choisie parmi par exemple parmi la cire d'abeille, les cires de paraffine, l'huile de ricin hydrogénée, les cires de silicone.

La cire additionnelle peut être présente sous forme de microdispersion de cire telle que décrite plus haut pour la première cire ou la deuxième cire.

La cire additionnelle peut être présente dans la composition selon l'invention en une teneur allant de 0,1 % à 50 % en poids, par rapport au poids total de la composition, de préférence de 0,5 % à 30 % en poids, et mieux de 1 % à 20 % en poids.

La teneur totale en cires (première cire et/ou seconde cire et/ou cire additionnelle) de la composition selon l'invention peut aller de 5 à 70% en poids par rapport au poids total de la composition, de préférence de 10 à 60%, et mieux de 15 à 50% en poids.

La composition selon l'invention peut comprendre au moins un composé gras pâteux à température ambiante. Par "corps gras pâteux" au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le corps gras pâteux peut être présent dans la composition selon l'invention en une teneur allant de 0,01 à 60% en poids, par rapport au poids total de la composition, de préférence allant de 0,5 à 45 % en poids, et mieux allant de 2 % à 30 % en poids, dans la composition.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges.
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition selon l'invention peut comprendre au moins un polymère filmogène.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C₁₋C₂₀, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle. Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyétherpolyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools.

Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique. On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être un polymère hydrosoluble et peut être présent dans une phase aqueuse de la composition ; le polymère est dons solubilisé dans la phase aqueuse de la composition. Comme exemples de polymères filmogènes hydrosolubles, on peut citer :
- les protéines comme les protéines d'origine végétale telles que les protéines de blé, de soja; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
   - les gommes arabiques, la gomme de guar, les dérivés du xanthane, la gomme de karaya ;
   - les alginates et les carraghénanes ;
   - les glycoaminoglycanes, l'acide hyaluronique et ses dérivés ;
   - la résine shellac, la gomme de sandaraque, les dammars, les élémis, les copals ;
   - l'acide désoxyribonucléïque ;
   - les muccopolysaccharides tels les chondroïtines sulfate,
et leurs mélanges.

Selon une autre variante de réalisation de la composition selon l'invention, le polymère filmogène peut être un polymère solubilisé dans une phase grasse liquide comprenant des huiles ou solvants organiques tels que ceux décrits précédemment (on dit alors que le polymère filmogène est un polymère liposoluble). Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides à température ambiante, appelés aussi huiles, généralement compatibles entre eux. De préférence, la phase grasse liquide comprend une huile volatile, éventuellement en mélange avec une huile non volatile, les huiles pouvant être choisies parmi les huiles citées précédemment.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de la copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères dee polystéarate de vinyle, polystéarate de vinyle réticulé à l'aide de divinylbenzène, de diallyléther ou de phtalate de diallyle, poly(méth)acrylate de stéaryle, polylaurate de vinyle, poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂₋C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.
Comme dispersion aqueuse de polymère filmogène, on peut utiliser les dispersions acryliques vendues sous les dénominations « Neocryl XK-90® », « Neocryl A-1070® », « Neocryl A-1090® », « Neocryl BT-62® », « Neocryl A-1079® » et « Neocryl A-523® » par la société AVECIA-NEORESINS, « Dow Latex 432® » par la société DOW CHEMICAL, « Daitosol 5000 AD® » par la société DAITO KASEY KOGYO; ou encore les dispersions aqueuses de polyuréthane vendues sous les dénominations « Neorez R-981® » et « Neorez R-974® » par la société AVECIA-NEORESINS, les «Avalure UR-405® », «Avalure UR-410® », «Avalure UR-425® », « Avalure UR-450® », « Sancure 875® », « Sancure 861® », « Sancure 878® » et « Sancure 2060® » par la société GOODRICH, « Impranil 85® » par la société BAYER, « Aquamere H-1511® » par la société HYDROMER ; les sulfopolyesters vendus sous le nom de marque « Eastman AQ® » par la société EASTMAN CHEMICAL PRODUCTS, les dispersions vinyliques comme le « Mexomère PAM » et aussi les dispersions acryliques dans l'isododécane comme le « Mexomère PAP » par la société CHIMEX.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

### Additifs

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition de l'invention peut comprendre, en outre, tout additif usuellement utilisé en cosmétique tels que les antioxydants, les charges, les conservateurs, les parfums, les neutralisants, les épaississants, les vitamines, les plastifiants et leurs mélanges.

Selon un mode de réalisation particulier de l'invention, la composition ne contient pas de filtre UV (filtre organique ou filtre minéral; filtre absorbant ou réfléchissant le rayonnement ultra-violet).

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique.

De préférence, la composition selon l'invention est une composition de revêtement des fibres kératiniques, et plus particulièrement un mascara, qui peut être conditionnée dans un produit d'application comprenant un réservoir et un moyen amovible pour fermer, de préférence de manière étanche, ledit réservoir. Ledit ensemble d'application peut comprendre en outre un organe d'application de la composition de maquillage sur les fibres kératiniques, et notamment les cils, ledit organe d'application permettant le prélèvement de la composition et la restitution de la composition prélevée sur les cils. Cet organe d'application est de préférence solidaire des moyens de fermeture étanche de l'ensemble. L'ensemble d'application peut également comprendre un organe d'essorage (ou essoreur) dudit organe d'application, l'organe d'essorage pouvant être solidaire du réservoir.

L'organe d'application peut être de préférence une brosse à mascara bien connue de l'homme du métier. Une telle brosse comprend notamment des poils disposés radialement autour d'une âme torsadée, en particulier une âme métallique. La brosse peut être de forme variée et comporter des découpes. Des brosses à mascara sont par exemple décrites dans les documents FR-A-2607373, EP-A-611170, EP-A-811336, EP-A-811337, EP-A-842620.

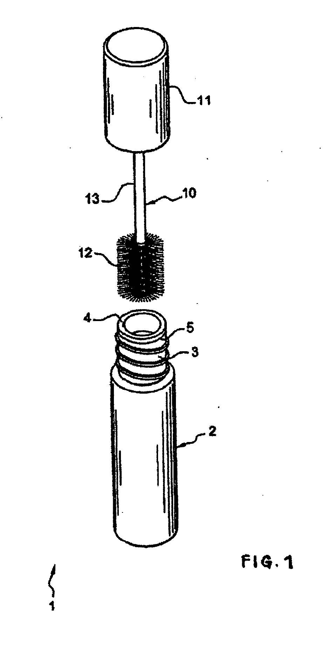

La figure 1 à laquelle il est maintenant fait référence représente un mode de réalisation préférentiel d'un ensemble de conditionnement et d'application 1 contenant une composition de mascara selon l'invention.

L'ensemble de conditionnement et d'application 1 comprend un récipient 2 surmonté d'un col fileté 3 dont un bord libre délimite une ouverture 4. Dans l'ouverture 4, est monté un organe d'essorage 5. L'ensemble 1 comprend également un dispositif d'application 10 comprenant un bouchon 11 solidaire d'une tige 13 dont une extrémité comporte un applicateur 12, configuré généralement sous forme d'un arrangement de fibres maintenues entre les deux branches d'un fil de fer torsadé. Une surface intérieure du bouchon 11 est filetée de manière à coopérer avec le filetage du col 3. Ainsi, lorsque l'applicateur 12 et la tige 13 sont disposés à l'intérieur du récipient 2, le filetage du bouchon 11 vient en engagement avec le filetage du col 3 de manière à ce que le bouchon obture de manière étanche l'ouverture 4 du récipient.

Alternativement, l'applicateur peut être constitué d'un peigne comprenant généralement une pluralité de dents obtenues de moulage avec un support en matériau thermoplastique. L'applicateur peut encore être constitué d'un peigne combiné avec une brosse.

L'invention est illustrée plus en détail dans les exemples suivants.

### Exemples 1 à 6 :

On a préparé un mascara anhydre selon l'invention (exemple 1) et 5 mascaras ne faisant pas partie de l'invention (exemples 2 à 6) ayant la composition suivante, en utilisant 6 cires différentes :
- Cire 27 g
- Bentonite 5,3 g
- Carbonate de propylène 1,7 g
- Copolymère acétate de vinyle/stéarate d'allyle (65/35)
   (Mexomère PQ de CHIMEX) 2,2 g
- Polylaurate de vinyle (Mexomère PP de CHIMEX) 0,7 g
- Silice ,8 g
- Pigments 3,6 g
- Conservateurs qs
- Isododécane qsp 100 g

Pour chaque composition, on a mesuré la viscosité et l'indice de consistance, et on a évalué la stabilité à 25 °C.

La mesure de la viscosité est effectuée à 25 °C avec un viscosimètre RHEOMAT RM 180 équipé d'un mobile n° 4, la mesure étant effectuée après 10 minutes de rotation du mobile (temps au bout duquel on observe une stabilisation de la viscosité et de la vitesse de rotation du mobile), à un cisaillement de 200 s⁻¹.

La mesure de l'indice de consistance est effectuée avec un texturomètre TA-TX2i par la Société RHEO, équipé d'une sonde cylindrique en inox de 12 mm de diamètre.

On remplit un récipient cylindrique (diamètre de 35 mm et profondeur de 15 mm) avec la composition de mascara à tester puis on arase la surface du produit contenu dans le récipient pour obtenir une surface bien plane du produit. La sonde cylindrique du texturomètre est déplacée à la vitesse de 10 mm.s⁻¹ puis pénètre dans le mascara contenu dans le récipient cylindrique jusqu'à une profondeur de 0,2 mm. On mesure alors la force exercée par le mascara sur la sonde, cette force correspondant à l'indice de consistance du mascara, exprimée en Pa.

La stabilité est évaluée par observation visuelle de la composition après deux semaines de stockage à 25 °C.

La mesure du collant et de la dureté de la cire est effectuée selon la méthode de mesure décrite précédemment dans la description.

On a obtenu les résultats suivants :

| **Exemple** | **Cire** | **Collant (N.s)** | **Dureté (MPa)** | **Viscosité (Pa.s)** | **Consistance (Pa)** | **Stabilité** |
|---|---|---|---|---|---|---|
| 1 | Koster K 82 P | 3,38 | 0,96 | 3,6 | 560 | oui |
| 2 | Abeille | 2,02 | 3,68 | 5,9 | 1842 | oui |
| 3 | Huile jojoba hydrogénée | 0,18 | 8,62 | 12,8 | 1991 | oui |
| 4 | Huile ricin hydrogénée | 0,08 | 2,77 | Trop épais | 22942 | - |
| 5 | Cire d'orange⁽¹⁾ | 0,09 | 0,09 | < 1 | Trop fluide | Non 2 phases |
| 6 | Cire de lanoline oxypropylénée (50P) ⁽²⁾ | 0,14 | 0,06 | < 1 | Trop fluide | Non 2 phases |

| | | | | | | |
|---|---|---|---|---|---|---|
| (1) vendue par la société Koster Keunen | | | | | | |
| (2) EMERY 1695 de la société COGNIS | | | | | | |

On constate que la composition 1 selon l'invention est stable et présente la plus faible viscosité et la plus faible consistance. Les compositions 2 et 3 bien que stables ont une viscosité et une consistance plus élevée que clles de la composition 1. La composition 4 est trop épaisse et n'est donc pas adaptée pour être appliquée sur les cils à l'aide d'une brosse à mascara.
Les compositions 5 et 6 ne sont pas stables : elles présentent 2 phases au bout de deux semaines de stockage à 25 °C.

### Exemple 7 :

On a préparé un mascara émulsion cire-dans-eau ayant la composition suivante :
- Cire collante (Kester Wax K 82 P de la société Koster Keunen) 28 g
- Amino-2 méthyl-2 propanediol-1,3 0,5 g
- Triéthanolamine 2,4 g
- Acide stéarique 5,8 g
- Polymères non-ioniques hydrosolubles 4,3 g
- Polyméthacrylate de sodium (Darvan 7 de la société VANDERBILT) 0,25 g MA
- Hydroxyéthylcellulose réticulée par l'épichlorhydrine quaternisée par la triméthylamine (JR 400 de la société UNION CARBIDE) 0,1 g
- Pigments 5,4 g
- Conservateurs qs
- Eau qsp 100g

Ce mascara est stable après 24 heures à température ambiante. Il s'applique facilement et adhère bien sur les cils. Le mascara forme un maquillage lisse et homogène, et épaissit les cils.

### Exemple 8:

On a préparé un mascara anhydre ayant la composition suivante :
- Cire collante (Kester Wax K 82 P de la société Koster Keunen) 30 g
- Bentonite 5,3 g
- Carbonate de propylène 1,7 g
- Copolymère acétate de vinyle/stéarate d'allyle (65/35)
   (Mexomère PQ de CHIMEX) 2,2 g
- Polylaurate de vinyle (Mexomère PP de CHIMEX) 0,7 g
- Stéarate de l'oligomère de l'acide 12-hydroxystéarique
   (SOLSPERSE 21000 de AVECIA) 0,1 g
- Talc 0,8 g
- Pigments 4,2 g
- Conservateurs qs
- Isododécane qsp 100 g

Ce mascara waterproof adhère bien sur les cils. Il confère aux cils un maquillage lisse et homogène, très séparant.

### Exemple 9 :

On a préparé un mascara anhydre ayant la composition suivante :
- Cire collante (Kester Wax K 82 P de la société Koster Keunen) 35 g
- Bentonite 5,3 g
- Carbonate de propylène 1,7 g
- Copolymère acétate de vinyle/stéarate d'allyle (65/35)
   (Mexomère PQ de CHIMEX) 2,2 g
- Polylaurate de vinyle (Mexomère PP de CHIMEX) 0,7 g
- Stéarate de l'oligomère de l'acide 12-hydroxystéarique
   (SOLSPERSE 21000 de AVECIA) 0,1 g
- Talc 0,8 g
- Pigments 4,2 g
- Conservateurs qs
- Isododécane qsp 100g

Ce mascara waterproof adhère bien sur les cils. Il confère aux cils un maquillage lisse et homogène et chargeant.

### Exemple 10 :

On a préparé un mascara émulsion cire-dans-eau ayant la composition suivante :
- Cire collante (Kester Wax K 82 P de la société Koster Keunen) 27 g
- Cire de carnauba 3 g
- Amino-2 méthyl-2 propanediol-1,3 0,5 g
- Triéthanolamine 2,4 g
- Acide stéarique 5,8 g
- Polymères non-ioniques hydrosolubles 4,3 g
- Polyméthacrylate de sodium (Darvan 7 de la société VANDERBILT) 0,25 g MA
- Hydroxyéthylcellulose réticulée par l'épichlorhydrine quaternisée par la triméthylamine (JR 400 de la société UNION CARBIDE) 0,1 g
- Pigments 5,4 g
- Conservateurs qs
- Eau qsp 100 g

Ce mascara est stable après 24 heures à température ambiante. Il s'applique facilement et adhère bien sur les cils. Le mascara forme un maquillage lisse et homogène, et épaissit les cils.

### Exemples 11 à 13

Les mascaras anhydres suivants ont été préparés :

| | | |
|---|---|---|
| Cire collante (Kester Wax K 82 P® de la société Koster Keunen) | | 27 g |
| Bentonite | | 2,66 g |
| Carbonate de propylène | | 1,7 g |
| Copolymère acétate de vinyle/stéarate d'allyle (65/35) | | |
| (Mexomère PQ® de CHIMEX) | | 2,2 g |
| Polylaurate de vinyle (Mexomère PP® de CHIMEX) | | 0,7 g |
| Stéarate de l'oligomère de l'acide 12-hydroxystéarique | | |
| (SOLSPERSE 21000® de AVECIA) | | 0,1 g |
| charge à surface spécifique | | 15 g |
| Pigments | | 4,2 g |
| Conservateurs | qs | |
| Isododécane | qsp | 100 g |

A partir de cette formulation, ont été préparés trois mascaras différents conformes à l'invention en incorporant pour chacun d'entre eux une charge spécifique.
La nature des trois charges choisies, leurs spécificités et quantités respectives sont indiquées dans le tableau 1.
La silice Sunsil 130® est commercialisé par Sunjin Chemical. Le polyméthacrylate de méthyle Jurymer MB1® est commercialisé par Nihon Junyakuet la silice Sunsphère H-51® est commercialisé par Asahi Glass.
Le collant est apprécié selon le protocole suivant :
On maquille une éprouvette constituée de cheveux raides (60 cheveux de 15mm de longueur) en appliquant le produit selon 30 passages successifs avec une brosse. Après un séchage d'une heure, on frotte les cheveux maquillés par un mouvement d'aller et retour avec le doigt. Le collant est apprécié qualitativement selon le degré de cheveux collés de 1 (pas collé du tout) à 5 (très collés).
Les trois mascaras waterproof ainsi obtenus adhèrent bien sur les cils. Ils confèrent aux cils un maquillage lisse et homogène, très séparant.

### Exemple 14

On a préparé un mascara anhydre waterproof ayant la composition suivante :

| | | |
|---|---|---|
| Cire collante (Kester Wax K 82 P® de la société Koster Keunen) | | 32 g |
| Palmitate de dextrine (Rheopearl KL® de Chiba Flour) | | 5,3 g |
| Copolymère acétate de vinyle/stéarate d'allyle (65/35) | | 2,2 g |
| (Mexomère PQ® de CHIMEX) | | |
| Polylaurate de vinyle (Mexomère PP® de CHIMEX) | | 0,75 g |
| Stéarate de l'oligomère de l'acide 12-hydroxystéarique | | 0,1 g |
| (SOLSPERSE 21000® de AVECIA) | | |
| Silice | | 10 g |
| Talc | | 0,84 g |
| Pigments | | 4,6 g |
| Conservateurs | qs | |
| Isododécane | qsp | 100 g |

Le mascara s'applique facilement sur les cils et permet d'obtenir un maquillage épais des cils et non collant : les cils sont bien séparés.

### Exemple 15

On a préparé un mascara émulsion cire / eau ayant la composition suivante :

| | | |
|---|---|---|
| Cire collante (Kester Wax K 82 P® de la société Koster Keunen) | | 27 g |
| Cire candellila | | 5 g |
| Palmitate de dextrine (Rheopearl KL® de Chiba Flour) | | 6 g |
| acide stéarique | | 5,8 g |
| amino-2 methyl-2 propanediol 1,3 | | 0,5 g |
| triethanolamine | | 2,4 g |
| hydroxyethycellulose 0,9 g | | |
| Silice | | 5 g |
| Pigments | | 5,5 g |
| Conservateurs | qs | |
| Eau | qsp | 100 g |

Le mascara s'applique très facilement sur les cils et permet d'obtenir un dépôt homogène, chargeant et séparant.

**Tableau 1**

| Exemple (Formule) | Nom commercial | Nature chimique | Masse (g) | Taille de la charge µm | Surface spécifique m²/g | Collant après frottement sur éprouvettes |
|---|---|---|---|---|---|---|
| N° 1 | Sunsil 130® | Silice creuse et sphérique | 15 | 6 - 9 | 200 - 260 | 1 |
| N° 2 | Jurymer MB1® | polyméthacrylate de méthyle | 15 | 8-15 | 300 | 2 |
| N°3 | Sunsphère® H-51 | Microsphères de silice creuse | 15 | 5 | 770 | 1 |

## Revendications

1. Composition de maquillage ou de soin des matières kératiniques comprenant, dans un milieu cosmétiquement acceptable, au moins 27% en poids, par rapport au poids total de la composition, d'une première cire ayant un collant supérieur ou égal à 0,7 N.s et une dureté inférieure ou égale à 3,5 MPa.

2. Composition selon la revendication 1 ou 2, **caractérisée par le fait que** la première cire a un collant allant de 0,7 N.s à 30 N.s.

3. Composition selon la revendication 1 ou 2, **caractérisée par le fait que** la première cire a un collant supérieur ou égal à 1 N.s, de préférence allant de 1 N.s à 20 N.s.

4. Composition selon l'une quelconque des revendications précédentes, **caractérisée par le fait que** la première cire a un collant supérieur ou égal à 2 N.s, de préférence allant de 2 N.s. à 10 N.s.

5. Composition selon l'une quelconque des revendications précédentes, **caractérisée par le fait que** la première cire a une dureté allant de 0,01 à 3,5 MPa.

6. Composition selon l'une quelconque des revendications précédentes, **caractérisée par le fait que** la première cire a une dureté allant de 0,05 MPa à 3 MPa, et de préférence allant de 0,1 MPa à 2,5 MPa.

7. Composition selon l'une quelconque des revendications précédentes, **caractérisée par le fait que** la première cire est un (hydroxystéaroyloxy)stéarate d'alkyle en C₂₀-C₄₀.

8. Composition selon la revendication 7, **caractérisée par le fait que** l'(hydroxystéaroyloxy)stéarate d'alkyle en C₂₀-C₄₀ répond à la formule (I) suivante : 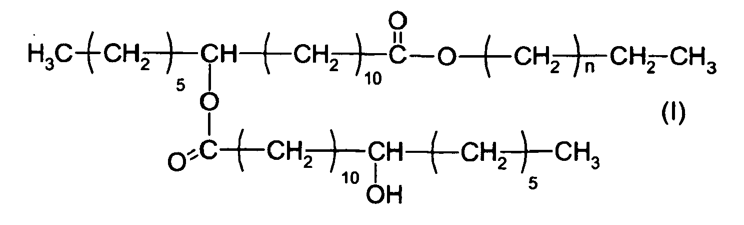 dans laquelle n est un entier allant de 18 à 38, ou un mélange de composés de formule (I).

9. Composition selon l'une des revendications précédentes, **caractérisée par le fait que** la première cire est présente en une teneur allant de 27 % à 60 % en poids, par rapport au poids total de la composition, de préférence allant de 28 % à 50 % en poids, et préférentiellement allant de 30 % à 40 % en poids.

10. Composition selon l'une quelconque des revendications précédentes, **caractérisée par le fait qu'**elle comprend une deuxième cire ayant une dureté supérieure ou égale à 6 MPa.

11. Composition selon la revendication précédente, **caractérisée par le fait que** la deuxième cire a une dureté allant de 6 MPa à 30 MPa, de préférence allant de 7 MPa à 25 MPa, préférentiellement allant de 8 MPa à 25 MPa, mieux allant de 9 à 20 MPa et encore mieux allant de 10 MPa à 20 MPa.

12. Composition selon la revendication 10 ou 11, **caractérisée par le fait que** la deuxième cire est choisie parmi la cire de carnauba, les cires de polyéthylène, la cire de Candelilla, l'huile de jojoba hydrogénée, le tétrastéarate de di-(triméthylol-1, 1, 1 propane), la cire obtenue par hydrogénation d'huile d'olive estérifiée avec l'alcool stéarylique.

13. Composition selon l'une des revendications 10 à 12, **caractérisée par le fait que** la deuxième cire est présente en une teneur allant de 0,1 % à 30 % en poids, par rapport au poids total de la composition, de préférence allant de 1 % à 20 % en poids, et plus préférentiellement allant de 2 % à 10 % en poids.

14. Composition selon l'une des revendications 1 à 13 **caractérisée en ce qu'**elle comprend au moins un composé choisi parmi un ester de dextrine et d'acide(s) gras et/ou une charge possédant une surface spécifique BET supérieure ou égale à 100 m²/g.

15. Composition selon la revendication 14, **caractérisée en ce que** l'ester de dextrine et d'acide(s) gras répond à la formule (II) : 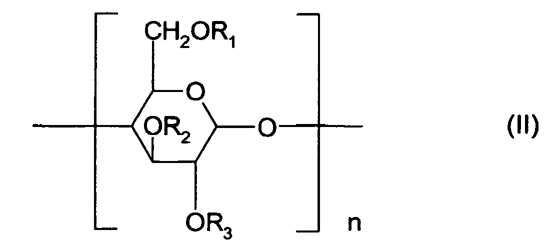 dans laquelle :
n est un entier allant de 3 à 200, notamment allant de 20 à 150, et en particulier allant de 25 à 50,
les radicaux R₁, R₂ et R₃, identiques ou différents, sont choisis parmi l'hydrogène ou un groupement acyle (R-CO-) dans lequel le radical R est un groupement hydrocarboné, linéaire ou ramifié, saturé ou insaturé, possédant de 7 à 29 atomes de carbone sous réserve qu'au moins un desdits radicaux R₁, R₂ ou R₃ est différent de l'hydrogène.

16. Composition selon la revendication 15, **caractérisée en ce qu'**au moins deux desdits radicaux R₁, R₂ ou R₃ sont identiques et différents de l'hydrogène.

17. Composition selon la revendication 15 ou 16, **caractérisée en ce que** l'ester de dextrine et d'acide gras a un degré de substitution inférieur ou égal à 2,5 sur la base d'une unité glucose, notamment variant de 1,5 à 2,5, et en particulier de 2 à 2,5.

18. Composition selon la revendication 15 ou 16, **caractérisée en ce que** n varie de 25 à 50 et notamment est égal à 38 dans la formule (II).

19. Composition selon l'une quelconque des revendications 15 à 18, **caractérisée en ce que** le groupement acyle est choisi parmi les radicaux caprylyle, caproyle, lauroyle, myristyle, palmityle, stéaryle, eicosanyle, docosanoyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécénoyle, palmitoléyle, oléyle, élaidyle, eicosényle, sorbyle, linoléyle, linolényle, punicyle, arachidonyle, stéarolyle, et leurs mélanges.

20. Composition selon l'une quelconque des revendications 15 à 19, **caractérisée en ce que** l'ester de dextrine et d'acide(s) gras comprend au moins le palmitate de dextrine.

21. Composition selon l'une quelconque des revendications 15 à 20, **caractérisée en ce que** le poids moléculaire moyen en poids de l'ester de dextrine et d'acide(s) gras varie de 10 000 à 150 000, notamment de 12 000 à 100 000, voire de 15 000 à 80 000.

22. Composition selon l'une quelconque des 15 à 21, **caractérisée en ce que** ledit ester de dextrine et d'acide(s) gras est présent à une quantité allant de 0,1 à 20 %, en particulier de 0,5 à 15 % en poids et notamment de 1 à 10 % en poids par rapport au poids total de la composition.

23. Composition selon l'une quelconque des revendications 15 à 22, **caractérisée en ce que** l'ester de dextrine et d'acide(s) gras et la cire collante sont présents à une teneur telle que le rapport pondéral de la cire collante par rapport audit ester varie de 350 à 0,1, en particulier de 100 à 0,5, notamment de 50 à 1, voire de 15 à 2.

24. Composition selon l'une quelconque des revendications 15 à 23, **caractérisée en ce que** l'ester de dextrine et d'acide(s) gras et l'(hydroxystearyloxy)stéarate d'alkyle sont présents en une teneur telle que le rapport pondéral de la cire collante par rapport audit ester varie de 350 à 0,1, en particulier de 100 à 0,5, notamment de 50 à 1, voire de 15 à 2.

25. Composition selon l'une des revendications 14 à 24, **caractérisée en ce qu'**elle comprend au moins une charge possédant une surface spécifique variant de 100 à 5000 m²/g notamment de 150 à 1000 m²/g, et en particulier de 200 à 800 m²/g.

26. Composition selon la revendication 14 ou 25, **caractérisée en ce que** la charge à surface spécifique est choisie parmi les charges organiques, minérales, et leurs mélanges.

27. Composition selon la revendication 26, **caractérisée en ce que** la charge à sur face spécifique organique est choisie parmi les cires de polyoléfines et notamment les cires de polyéthylène et les charges polymériques du type polyméthylméthacrylate ou poytétrafluoroéthylène.

28. Composition selon la revendication 26, **caractérisée en ce que** la charge à surface spécifique minérale est choisie parmi les silices, les alumines, les silicates, et les aluminosilicates.

29. Composition selon la revendication 14 ou l'une des revendications 25 à 28, **caractérisée en ce que** les particules composant la charge ont une taille moyenne variant de 0,01 à 100 µm, notamment de 0,1 à 50 µm et en particulier de 1 à 20 µm.

30. Composition selon la revendication 14 ou l'une des revendications 25 à 29, **caractérisée en ce que** les particules composant la charge à surface spécifique sont creuses.

31. Composition selon la revendication 14 ou l'une des revendications 25 à 30, **caractérisée en ce que** les particules composant la charge à surface spécifique sont des microsphères de silice creuse.

32. Composition selon la revendication 14 ou l'une des revendications 25 à 31, **caractérisée en ce que** le rapport pondéral de la cire collante par rapport à la charge à surface spécifique varie de 350 à 0,1, notamment de 100 à 0,5, et en particulier de 50 à 0,8 et mieux de 30 à 1.

33. Composition selon la revendication 14 ou l'une des revendications 25 à 32, **caractérisée en ce qu'**elle contient de 0,1 à 25 % de charge à surface spécifique, notamment de 0,5 à 20 %, et en particulier de 1 à 15 % en poids par rapport au poids total de la composition.

34. Composition selon l'une quelconque des revendications précédentes, **caractérisée par le fait qu'**elle comprend une phase aqueuse.

35. Composition selon l'une quelconque des revendications précédentes, **caractérisée par le fait qu'**elle comprend une phase aqueuse formée d'eau ou d'un mélange d'eau et de solvant organique miscible à l'eau.

36. Composition selon la revendication précédente, **caractérisée par le fait que** le solvant organique miscible à l'eau est choisi parmi les monoalcools inférieurs ayant de 1 à 5 atomes de carbone, les glycols ayant de 2 à 8 atomes de carbone, les cétones en C₃-C₄, les aldéhydes en C₂-C₄.

37. Composition selon la revendication 35 ou 36, **caractérisée par le fait que** la phase aqueuse est présente en une teneur allant de 1 % à 95 % en poids, par rapport au poids total de la composition, de préférence allant de 3 % à 80 % en poids, et préférentiellement allant de 5 % à 60 % en poids.

38. Composition selon l'une quelconque des revendications précédentes, **caractérisée par le fait qu'**elle comprend une huile volatile.

39. Composition selon la revendication précédente, **caractérisée par le fait que** l'huile volatile est choisies parmi les huiles hydrocarbonées, les huiles siliconées, ou leurs mélanges.

40. Composition selon l'une des revendications 38 ou 39 **caractérisée par le fait que** l'huile volatile est présente en une teneur allant de 0,1 % à 98 % en poids, par rapport au poids total de la composition, de préférence allant de 1 % à 65 % en poids.

41. Composition selon l'une quelconque des revendications précédentes, **caractérisée par le fait qu'**elle comprend une huile non volatile.

42. Composition selon la revendication précédente, **caractérisée par le fait que** l'huile non volatile est présente en une teneur allant de 0,1 % à 50 % en poids, de préférence de 0,1 % à 40 % en poids, par rapport au poids total de la composition, et mieux de 0,1 % à 30 % en poids.

43. Composition selon l'une quelconque des revendications précédentes, **caractérisée par le fait qu'**elle comprend un polymère filmogène.

44. Composition selon la revendication 43, **caractérisée par le fait que** le polymère filmogène est présent en une teneur en matières sèches allant de 0,1 % à 60 % en poids par rapport au poids total de la composition, de préférence allant de 0,5 % à 40 % en poids, et préférentiellement allant de 1 % à 30 % en poids.

45. Composition selon l'une quelconque des revendications précédentes, **caractérisée par le fait qu'**elle comprend une cire additionnelle.

46. Composition selon la revendication précédente, **caractérisée par le fait que** la cire additionnelle est présente en une teneur allant de 0,1 % à 50% en poids, par rapport au poids total de la composition, de préférence de 0,5% à 30 % en poids, et mieux de 1 % à 20 % en poids.

47. Composition selon l'une quelconque des revendications précédentes, **caractérisée par le fait qu'**elle comprend un tensioactif.

48. Composition selon l'une quelconque des revendications précédentes, caracté-risée par le fait qu'elle comprend un additif choisi parmi les matières colorantes, les antioxydants, les charges, les corps gras pâteux, les conservateurs, les parfums, les neutralisants, les épaississants, les vitamines, les plastifiants, et leurs mélanges.

49. Composition selon l'une quelconque des revendications précédentes, **caractérisée par le fait qu'**elle ne contient pas de filtre UV.

50. Composition selon l'une quelconque des revendications précédentes, **caractérisée par le fait qu'**elle se présente sous la forme de mascara, de produit pour les sourcils, d'eye-liner, de fard à paupières, de fard à joue, de fond de teint, de produit pour les lèvres, de produit de maquillage du corps (tatouage semi-permanent), de produit de maquillage des cheveux.

51. Composition selon l'une quelconque des revendications précédentes, caracté-risée par le fait qu'elle se présente sous la forme de mascara.

52. Composition selon l'une quelconque des revendications précédentes, **caractérisée par le fait qu'**elle se présente sous la forme, d'eye-liner, de fard à paupières, de fard à joue, de fond de teint, de produit pour les lèvres, de produit de maquil-lage du corps (tatouage semi-permanent).

53. Composition selon l'une quelconque des revendications précédentes, **caractérisée par le fait qu'**elle est une composition de soin ou de maquillage des fibres kératiniques.

54. Composition selon l'une quelconque des revendications précédentes, **caractérisée par le fait qu'**elle est un mascara.

55. Procédé cosmétique de maquillage ou de soin non thérapeutique des matières kératiniques comprenant l'application sur les matières kératiniques d'une composition selon l'une quelconque des revendications précédentes.

56. Utilisation d'une composition selon l'une quelconque des revendications 1 à 54 pour obtenir un maquillage des matières kératiniques homogène et/ou lisse et/ou un maquillage rapide des matières kératiniques.

57. Utilisation d'une composition selon l'une des revendications 14 à 54 pour obtenir un maquillage chargeant et séparant des cils.

58. Utilisation d'une cire ayant un collant supérieur ou égal à 0,7 N.s et une dureté inférieure ou égale à 3,5 MPa, dans une composition cosmétique,pour obtenir un maquillage des matières kératiniques rapide et/ou homogène et/ou lisse et/ou obtenir une composition cosmétique stable, ladite cire étant présente en une teneur d'au moins 27 % en poids, par rapport au poids total de la composition.

59. Utilisation d'au moins un composé choisi parmi un ester de dextrine et d'acide(s) gras et/ou une charge possédant une surface spécifique BET supérieure ou égale à 100 m²/g dans une composition cosmétique contenant au moins 27 % en poids, par rapport au poids total de la composition, d'au moins une cire ayant un collant supérieur ou égal à 0,7 N.s et une dureté inférieure ou égale à 3,5 MPa, pour obtenir un maquillage des matières kératiniques homogène et/ou lisse et notamment un maquillage des fibres kératiniques, chargeant et séparant et/ou obtenir une composition cosmétique stable.

60. Utilisation selon l'une des revendications 58 ou 59, **caractérisée par le fait que** la cire est un (hydroxystéaroyloxy)stéarate d'alkyle en C₂₀-C₄₀ répondant à la formule (1) suivante : 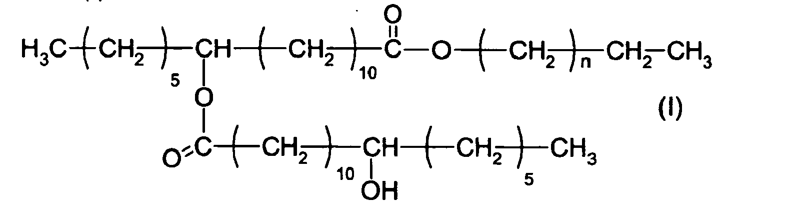 dans laquelle n est un entier allant de 18 à 38, ou un mélange de composés de formule (I).

61. Ensemble (1) de conditionnement et d'application d'un produit de revêtement des fibres kératiniques comprenant :
i) un récipient (2) contenant une composition selon l'une quelconque des revendications 1 à 53, et
ii) des moyens (12), notamment sous forme d'une brosse torsadée ou d'un peigne, pour l'application de la composition sur les fibres.

## Claims

1. Makeup or care composition for keratin materials comprising, in a cosmetically acceptable medium, at least 27% by weight, relative to the total weight of the composition, of a first wax having a tack of greater than or equal to 0.7 N.s and a hardness of less than or equal to 3.5 MPa.

2. Composition according to Claim 1 or 2, **characterized in that** the first wax has a tack ranging from 0.7 N.s to 30 N.s.

3. Composition according to Claim 1 or 2, **characterized in that** the first wax has a tack of greater than or equal to 1 N.s and preferably ranging from 1 N.s to 20 N.s.

4. Composition according to any one of the preceding claims, **characterized in that** the first wax has a tack of greater than or equal to 2 N.s and preferably ranging from 2 N.s to 10 N.s.

5. Composition according to any one of the preceding claims, **characterized in that** the first wax has a hardness ranging from 0.01 to 3.5 MPa.

6. Composition according to any one of the preceding claims, **characterized in that** the first wax has a hardness ranging from 0.05 MPa to 3 MPa and preferably ranging from 0.1 MPa to 2.5 MPa.

7. Composition according to any one of the preceding claims, **characterized in that** the first wax is a C₂₀-C₄₀ alkyl (hydroxystearoyloxy) stearate.

8. Composition according to Claim 7, **characterized in that** the C₂₀-C₄₀ alkyl (hydxoxystearoyloxy) stearate corresponds to formula (I) below: 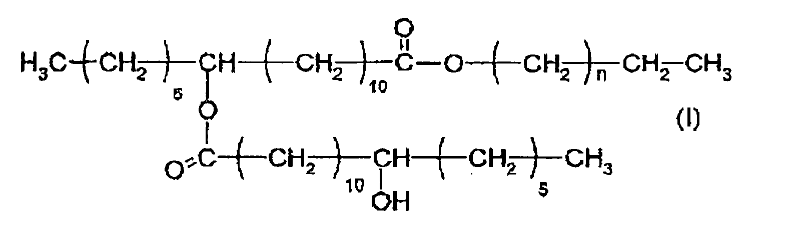 in which n is an integer ranging from 18 to 38, or a mixture of compounds of formula (I).

9. Composition according to one of the preceding claims, **characterized in that** the first wax is present in a content ranging from 27% to 60% by weight, preferably ranging from 28% to 50% by weight and preferentially ranging from 30% to 40% by weight, relative to the total weight of the composition.

10. Composition according to any one of the preceding claims, **characterized in that** it comprises a second wax with a hardness of greater than or equal to 6 MPa.

11. Composition according to the preceding claim, **characterized in that** the second wax has a hardness ranging from 6 MPa to 30 MPa, preferably ranging from 7 MPa to 25 MPa, preferentially ranging from 8 MPa to 25 MPa, better still ranging from 9 to 20 MPa and even better still ranging from 10 MPa to 20 MPa.

12. Composition according to Claim 10 or 11, **characterized in that** the second wax is chosen from carnauba wax, polyethylene waxes, candelilla wax, hydrogenated jojoba oil, bis(1,1,1-trimethylolpropane) tetrastearate and the wax obtained by hydrogenation of olive oil esterified with stearyl alcohol.

13. Composition according to one of Claims 10 to 12, **characterized in that** the second wax is present in a content ranging from 0.1% to 30% by weight, preferably ranging from 1% to 20% by weight and more preferably ranging from 2% to 10% by weight, relative to the total weight of the composition.

14. Composition according to one of Claims 1 to 13, **characterized in that** it comprises at least one compound chosen from a dextrin ester of fatty acid(s) and/or a filler having a BET specific surface area of greater than or equal to 100 m²/g.

15. Composition according to Claim 14, **characterized in that** the dextrin ester of fatty acid(s) corresponds to formula (II) : 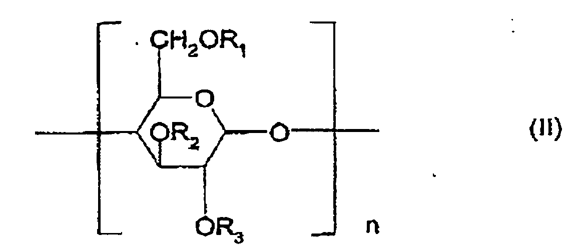 in which:
- n is an integer ranging from 3 to 200, especially ranging from 20 to 150 and in particular ranging from 25 to 50,
- the radicals R₁, R₂ and R₃, which may be identical or different, are chosen from hydrogen and an acyl group (R-CO-) in which the radical R is a linear or branched, saturated or unsaturated hydrocarbon-based group containing from 7 to 29 carbon atoms, with the proviso that at least one of the said radicals R₁, R₂ or R₃ is other than hydrogen.

16. Composition according to Claim 15, **characterized in that** at least two of the said radicals R₁, R₂ or R₃ are identical and other than hydrogen.

17. Composition according to Claim 15 or 16, **characterized in that** the dextrin ester of fatty acid has a degree of substitution of less than or equal to 2.5, especially ranging from 1.5 to 2.5 and in particular from 2 to 2.5, on the basis of one glucose unit.

18. Composition according to Claim 15 or 16, **characterized in that** n ranges from 25 to 50 and is especially equal to 38 in formula (II).

19. Composition according to any one of Claims 15 to 18, **characterized in that** the acyl group is chosen from caprylyl, caproyl, lauroyl, myristyl, palmityl, stearyl, eicosanyl, docosanoyl, isovaleryl, 2-ethylbutyryl, ethylmethylacetyl, isoheptanyl, 2-ethylhexanyl, isononanyl, isodecanyl, isotridecanyl, isomyristyl, isopalmityl, isostearyl, isohexanyl, decenyl, dodecenyl, tetradecenyl, myristyl, hexadecenoyl, palmitoleyl, oleyl, elaidyl, eicosenyl, sorbyl, linoleyl, linolenyl, punicyl, arachidonyl and stearolyl, radicals, and mixtures thereof.

20. Composition according to any one of Claims 15 to 19, **characterized in that** the dextrin ester of fatty acid(s) comprises at least dextrin palmitate.

21. Composition according to any one of Claims 15 to 20, **characterized in that** the weight-average molecular weight of the dextrin ester of fatty acid(s) ranges from 10 000 to 150 000 and especially from 12 000 to 100 000, or even from 15 000 to 80 000.

22. Composition according to any one of Claims 15 to 21, **characterized in that** the said dextrin ester of fatty acid(s) is present in an amount ranging from 0.1% to 20%, in particular from 0.5% to 15% by weight and especially from 1% to 10% by weight, relative to the total weight of the composition.

23. Composition according to any one of Claims 15 to 22, **characterized in that** the dextrin ester of fatty acid(s) and the tacky wax are present in a content such that the weight ratio of the tacky wax relative to the said ester ranges from 350 to 0.1, in particular from 100 to 0.5 and especially from 50 to 1, or even from 15 to 2.

24. Composition according to any one of Claims 15 to 23, **characterized in that** the dextrin ester of fatty acid(s) and the alkyl (hydroxystearyloxy)stearate are present in a content such that the weight ratio of the tacky wax relative to the said ester ranges from 350 to 0.1, in particular from 100 to 0.5 and especially from 50 to 1, or even from 15 to 2.

25. Composition according to one of Claims 14 to 24, **characterized in that** it comprises at least one filler having a specific surface area ranging from 100 to 5000 m²/g, especially from 150 to 1000 m²/g and in particular from 200 to 800 m²/g.

26. Composition according to Claim 14 or 25, **characterized in that** the filler with a specific surface area is chosen from organic and mineral fillers, and mixtures thereof.

27. Composition according to Claim 26, **characterized in that** the organic filler with a specific surface area is chosen from polyolefin waxes and especially polyethylene waxes, and polymer fillers of the polymethyl methacrylate or polytetrafluoroethylene type.

28. Composition according to Claim 26, **characterized in that** the mineral filler with a specific surface area is chosen from silicas, aluminas, silicates and aluminosilicates.

29. Composition according to Claim 14 or one of Claims 25 to 28, **characterized in that** the particles of which the filler is composed have a mean size ranging from 0.01 to 100 µm, especially from 0.1 to 50 µm and in particular from 1 to 20 µm.

30. Composition according to Claim 14 or one of Claims 25 to 29, **characterized in that** the particles of which the filler with a specific surface area is composed are hollow.

31. Composition according to Claim 14 or one of Claims 25 to 30, **characterized in that** the particles of which the filler with a specific surface area is composed are hollow silica microspheres.

32. Composition according to Claim 14 or one of Claims 25 to 31, **characterized in that** the weight ratio of the tacky wax relative to the filler with a specific surface area ranges from 350 to 0.1, especially from 100 to 0.5, in particular from 50 to 0.8 and better still from 30 to 1.

33. Composition according to Claim 14 or one of Claims 25 to 32, **characterized in that** it contains from 0.1% to 25% of filler with a specific surface area, especially from 0.5% to 20% and in particular from 1% to 15% by weight, relative to the total weight of the composition.

34. Composition according to any one of the preceding claims, **characterized in that** it comprises an aqueous phase.

35. Composition according to any one of the preceding claims, **characterized in that** it comprises an aqueous phase formed from water or from a mixture of water and water-miscible organic solvent.

36. Composition according to the preceding claim, **characterized in that** the water-miscible organic solvent is chosen from lower monoalcohols containing from 1 to 5 carbon atoms, glycols containing from 2 to 8 carbon atoms, C₃-C₄ ketones and C₂-C₄ aldehydes.

37. Composition according to Claim 35 or 36,
**characterized in that** the aqueous phase is present in a content ranging from 1% to 95% by weight, preferably ranging from 3% to 80% by weight and preferentially ranging from 5% to 60% by weight, relative to the total weight of the composition.

38. Composition according to any one of the preceding claims, **characterized in that** it comprises a volatile oil.

39. Composition according to the preceding claim, **characterized in that** the volatile oil is chosen from hydrocarbon-based oils and silicone oils, or mixtures thereof.

40. Composition according to either of Claims 38 and 39, **characterized in that** the volatile oil is present in a content ranging from 0.1% to 98% by weight and preferably ranging from 1% to 65% by weight relative to the total weight of the composition.

41. Composition according to any one of the preceding claims, **characterized in that** it comprises a non-volatile oil.

42. Composition according to the preceding claim, **characterized in that** the non-volatile oil is present in a content ranging from 0.1% to 50% by weight, preferably from 0.1% to 40% by weight and better still from 0.1% to 30% by weight, relative to the total weight of the composition.

43. Composition according to any one of the preceding claims, **characterized in that** it comprises a film-forming polymer.

44. Composition according to Claim 43, **characterized in that** the film-forming polymer is present in a solids content ranging from 0.1% to 60% by weight, preferably ranging from 0.5% to 40% by weight and preferentially ranging from 1% to 30% by weight, relative to the total weight of the composition.

45. Composition according to any one of the preceding claims, **characterized in that** it comprises an additional wax.

46. Composition according to the preceding claim, **characterized in that** the additional wax is present in a content ranging from 0.1% to 50% by weight, preferably from 0.5% to 30% by weight and better still from 1% to 20% by weight, relative to the total weight of the composition.

47. Composition according to any one of the preceding claims, **characterized in that** it comprises a surfactant.

48. Composition according to any one of the preceding claims, **characterized in that** it comprises an additive chosen from dyestuffs, antioxidants, fillers, pasty fatty substances, preserving agents, fragrances, neutralizers, thickeners, vitamins and plasticizers, and mixtures thereof.

49. Composition according to any one of the preceding claims, **characterized in that** it contains no UV-screening agent.

50. Composition according to any one of the preceding claims, **characterized in that** it is in the form of a mascara, an eyebrow product, an eyeliner, an eyeshadow, a makeup rouge, a foundation, a lip product, a body makeup product (semi-permanent tattoo) or a hair makeup product.

51. Composition according to any one of the preceding claims, **characterized in that** it is in the form of a mascara.

52. Composition according to any one of the preceding claims, **characterized in that** it is in the form of an eyeliner, an eyeshadow, a makeup rouge, a foundation, a lip product or a body makeup product (semi-permanent tattoo).

53. Composition according to any one of the preceding claims, **characterized in that** it is in the form of a care or makeup composition for keratin fibres.

54. Composition according to any one of the preceding claims, **characterized in that** it is a mascara.

55. Non-therapeutic cosmetic makeup or care process for keratin materials, comprising the application to the keratin materials of a composition according to any one of the preceding claims.

56. Use of a composition according to any one of Claims 1 to 54 to obtain a uniform and/or smooth makeup result on keratin materials and/or a rapid makeup result on keratin materials.

57. Use of a composition according to one of Claims 14 to 54, to obtain a charging and separating makeup result on the eyelashes.

58. Use of a wax having a tack of greater than or equal to 0.7 N.s and a hardness of less than or equal to 3.5 MPa, in a cosmetic composition, to obtain a rapid and/or uniform and/or smooth makeup result on keratin materials and/or to obtain a stable cosmetic composition, the said wax being present in a content of at least 27% by weight relative to the total weight of the composition.

59. Use of at least one compound chosen from a dextrin ester of fatty acid(s) and/or a filler having a BET specific surface area of greater than or equal to 100 m²/g in a cosmetic composition containing at least 27% by weight, relative to the total weight of the composition, of at least one wax having a tack of greater than or equal to 0.7 N.s and a hardness of less than or equal to 3.5 MPa, to obtain a uniform and/or smooth makeup result on keratin materials and especially a charging and separating makeup result on keratin fibres and/or to obtain a stable cosmetic composition.

60. Use according to either of Claims 58 and 59, **characterized in that** the wax is a C₂₀-C₄₀ alkyl (hydroxystearoyloxy)stearate corresponding to formula (I) below: 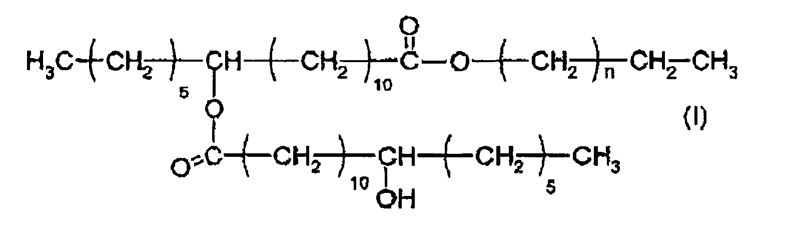 in which n is an integer ranging from 18 to 38, or a mixture of compounds of formula (I).

61. Assembly (1) for packaging and applying a product for coating keratin fibres, comprising:
i) a container (2) containing a composition according to any one of Claims 1 to 53, and
ii) means (12), especially in the form of a twisted brush or a comb, for applying the composition to the fibres.

## Patentansprüche

1. Zusammensetzung zum Schminken oder für die Pflege von Keratinsubstanzen, die in einem kosmetisch akzeptablen Medium, bezogen auf das Gesamtgewicht der Zusammensetzung, mindestens 27 Gew.-% eines ersten Wachses mit einer Klebrigkeit von 0,7 N·s oder darüber und einer Härte von 3,5 MPa oder darunter enthält.

2. Zusammensetzung nach Anspruch 1 oder 2, **dadurch gekennzeichnet, dass** das erste Wachs eine Klebrigkeit von 0,7 bis 30 N·s aufweist.

3. Zusammensetzung nach Anspruch 1 oder 2, **dadurch gekennzeichnet, dass** das erste Wachs eine Klebrigkeit von 1 N·s oder darüber und vorzugsweise 1 bis 20 N·s aufweist.

4. Zusammensetzung nach einem der vorhergehenden Ansprüche, **dadurch gekennzeichnet, dass** das erste Wachs eine Klebrigkeit von 2 N·s oder darüber und vorzugsweise 2 bis 10 N·s aufweist.

5. Zusammensetzung nach einem der vorhergehenden Ansprüche, **dadurch gekennzeichnet, dass** das erste Wachs eine Härte von 0,01 bis 3,5 MPa besitzt.

6. Zusammensetzung nach einem der vorhergehenden Ansprüche, **dadurch gekennzeichnet, dass** das erste Wachs eine Härte von 0,05 bis 3 MPa und vorzugsweise 0,1 bis 2,5 MPa aufweist.

7. Zusammensetzung nach einem der vorhergehenden Ansprüche, **dadurch gekennzeichnet, dass** das erste Wachs ein Alkyl-(hydroxystearoyloxy)stearat mit 20 bis 40 Kohlenstoffatomen ist.

8. Zusammensetzung nach Anspruch 7, **dadurch gekennzeichnet, dass** das Alkyl(C₂₀₋₄₀)(hydroxystearoyloxy)stearat der folgenden Formel (I) entspricht: 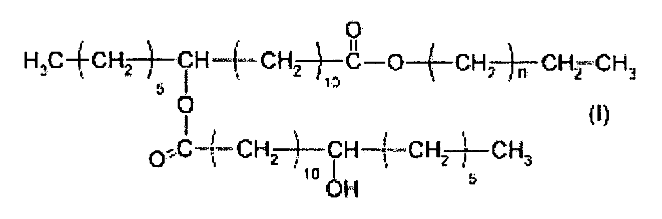 worin n eine ganze Zahl von 18 bis 38 bedeutet, oder einem Gemisch von Verbindungen der Formel (I).

9. Zusammensetzung nach einem der vorhergehenden Ansprüche, **dadurch gekennzeichnet, dass** das erste Wachs in einer Menge von 27 bis 60 Gew.-%, bezogen auf das Gesamtgewicht der Zusammensetzung, vorzugsweise 28 bis 50 Gew.-% und bevorzugt 30 bis 40 Gew.-% enthalten ist.

10. Zusammensetzung nach einem der vorhergehenden Ansprüche, **dadurch gekennzeichnet, dass** sie ein zweites Wachs mit einer Härte von 6 MPa oder darüber enthält.

11. Zusammensetzung nach dem vorhergehenden Anspruch, **dadurch gekennzeichnet, dass** das zweite Wachs eine Härte von 6 bis 30 MPa, vorzugsweise 7 bis 25 MPa, bevorzugt 8 bis 25 MPa, besser 9 bis 20 MPa und noch besser 10 bis 20 MPa aufweist.

12. Zusammensetzung nach Anspruch 10 oder 11, **dadurch gekennzeichnet, dass** das zweite Wachs unter Carnaubawachs, Polyethylenwachsen, Candilillawachs, hydriertem Jojobaöl, Di(1,1,1-trimethylolpropan)tetrastearat, durch Hydrierung von Olivenöl erhaltenes, mit Stearylalkohol verestertes Wachs.

13. Zusammensetzung nach einem der Ansprüche 10 bis 12, **dadurch gekennzeichnet, dass** das zweite Wachs in einer Menge von 0,1 bis 30 Gew.-%, bezogen auf das Gesamtgewicht der Zusammensetzung, vorzugsweise 1 bis 20 Gew.-% und noch bevorzugter 2 bis 10 Gew.-% enthalten ist.

14. Zusammensetzung nach einem der Ansprüche 1 bis 13, **dadurch gekennzeichnet, dass** sie mindestens eine Verbindung enthält, die unter einem Ester aus Dextrin und einer oder mehreren Fettsäuren und/oder einem Füllstoff mit einer spezifischen BET-Oberfläche von 100 m²/g oder darüber ausgewählt ist.

15. Zusammensetzung nach Anspruch 14, **dadurch gekennzeichnet, dass** der Ester aus Dextrin und Fettsäure(n) der Formel (II) entspricht: 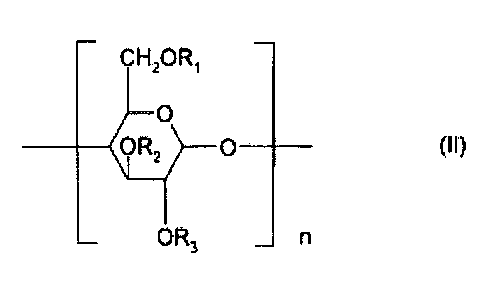 worin bedeuten:
n ist eine ganze Zahl von 3 bis 200, insbesondere 20 bis 150 und besonders 25 bis 50,
die Gruppen R₁, R₂ und R₃, die gleich oder verschieden sind, sind unter Wasserstoff oder einer Acylgruppe (R-CO-) ausgewählt, worin die Gruppe R eine geradkettige oder verzweigte, gesättigte oder ungesättigte Kohlenwasserstoffgruppe mit 7 bis 29 Kohlenstoffatomen ist, mit der Maßgabe, dass mindestens eine der Gruppen R₁, R₂ oder R₃ von Wasserstoff verschieden ist.

16. Zusammensetzung nach Anspruch 15, **dadurch gekennzeichnet, dass** mindestens zwei Gruppen R₁, R₂ oder R₃ identisch und von Wasserstoff verschieden sind.

17. Zusammensetzung nach Anspruch 15 oder 16, **dadurch gekennzeichnet, dass** der Dextrinfettsäureester einen Substitutionsgrad von höchstens 2,5 auf der Basis einer Glucoseeinheit und insbesondere im Bereich von 1,5 bis 2,5 und besonders 2 bis 2,5 aufweist.

18. Zusammensetzung nach Anspruch 15 oder 16, **dadurch gekennzeichnet, dass** in der Formel (II) n im Bereich von 25 bis 50 liegt und insbesondere 38 beträgt.

19. Zusammensetzung nach einem der Ansprüche 15 bis 18, **dadurch gekennzeichnet, dass** die Acylgruppe unter den Gruppen Caprylyl, Caproyl, Lauroyl, Myristyl, Palmityl, Stearyl, Eicosanyl, Docosanoyl, Isovaleryl, 2-Ethylbutyryl, Ethylmethylacetyl, Isoheptanyl, 2-Ethylhexanyl, Isononanyl, Isodecanyl, Isotridecanyl, Isomyristyl, Isopalmityl, Isostearyl, Isohexanyl, Decenyl, Dodecenyl, Tetradecenyl, Myristyl, Hexadecenoyl, Palmitoleyl, Oleyl, Elaidyl, Eicosenyl, Sorbyl, Linoleyl, Linolenyl, Punicyl, Arachidonyl, Stearoyl und deren Gemischen ausgewählt ist.

20. Zusammensetzung nach einem der Ansprüche 15 bis 19, **dadurch gekennzeichnet, dass** der Ester aus Dextrin und einer oder mehreren Fettsäuren zumindest das Dextrinpalmitat umfasst.

21. Zusammensetzung nach einem der Ansprüche 15 bis 20, **dadurch gekennzeichnet, dass** die gewichtsmittlere Molmasse des Esters aus Dextrin und Fettsäure(n) im Bereich von 10.000 bis 150.000, insbesondere 12.000 bis 100.000 und besonders 15.000 bis 80.000 liegt.

22. Zusammensetzung nach einem der Ansprüche 15 bis 21, **dadurch gekennzeichnet, dass** der Ester aus Dextrin und Fettsäure(n) in einer Menge von 0,1 bis 20 Gew.-%, besonders 0,5 bis 15 Gew.-% und insbesondere 1 bis 10 Gew.-%, bezogen auf das Gesamtgewicht der Zusammensetzung, enthalten ist.

23. Zusammensetzung nach einem der Ansprüche 15 bis 22, **dadurch gekennzeichnet, dass** der Ester aus Dextrin und Fettsäure(n) und das klebrige Wachs in einer solchen Menge enthalten sind, dass das Gewichtsverhältnis des klebrigen Wachses bezogen auf den Ester im Bereich von 350 bis 0,1, insbesondere 100 bis 0,5, besonders 50 bis 1 und speziell 15 bis 2 liegt.

24. Zusammensetzung nach einem der Ansprüche 15 bis 23, **dadurch gekennzeichnet, dass** der Ester aus Dextrin und Fettsäure(n) und Alkyl(hydroxystearyloxy)stearat in einer solchen Menge enthalten sind, dass das Gewichtsverhältnis des klebrigen Wachses bezogen auf den Ester im Bereich von 350 bis 0,1, insbesondere 100 bis 0,5, besonders 50 bis 1 und speziell 15 bis 2 liegt.

25. Zusammensetzung nach einem der Ansprüche 14 bis 24, **dadurch gekennzeichnet, dass** sie mindestens einen Füllstoff enthält, der eine spezifische Oberfläche besitzt, die im Bereich von 100 bis 5.000 m²/g, insbesondere 150 bis 1.000 m²/g und besonders 200 bis 800 m²/g liegt.

26. Zusammensetzung nach Anspruch 14 oder 25, **dadurch gekennzeichnet, dass** der Füllstoff mit spezifischer Oberfläche unter den organischen Füllstoffen, anorganischen Füllstoffen und deren Gemischen ausgewählt ist.

27. Zusammensetzung nach Anspruch 26, **dadurch gekennzeichnet, dass** der organische Füllstoff mit spezifischer Oberfläche unter den Polyolefinwachsen und besonders den Polyethylenwachsen und den polymeren Füllstoffen vom Typ Polymethylmethacrylat oder Polytetrafluorethylen ausgewählt ist.

28. Zusammensetzung nach Anspruch 26, **dadurch gekennzeichnet, dass** der anorganische Füllstoff mit spezifischer Oberfläche unter den Kieselsäuren, Aluminiumoxiden, Silicaten und Aluminosilicaten ausgewählt ist.

29. Zusammensetzung nach Anspruch 14 oder nach einem der Ansprüche 25 bis 28, **dadurch gekennzeichnet, dass** die Partikel, die den Füllstoff bilden, eine mittlere Größe von 0,01 bis 100 µm, insbesondere 0,1 bis 50 µm und besonders 1 bis 20 µm besitzen.

30. Zusammensetzung nach Anspruch 14 oder einem der Ansprüche 25 bis 29, **dadurch gekennzeichnet, dass** die Partikel, die den Füllstoff mit spezifischer Oberfläche bilden, hohl sind.

31. Zusammensetzung nach Anspruch 14 oder einem der Ansprüche 25 bis 30, **dadurch gekennzeichnet, dass** die Partikel, die den Füllstoff mit spezifischer Oberfläche bilden, Siliciumoxid-Mikrohohlkugeln sind.

32. Zusammensetzung nach Anspruch 14 oder einem der Ansprüche 25 bis 31, **dadurch gekennzeichnet, dass** das Gewichtsverhältnis des klebrigen Wachses bezogen auf den Füllstoff mit spezifischer Oberfläche im Bereich von 350 bis 0,1, insbesondere 100 bis 0,5 und besonders 50 bis 0,8 und besser 30 bis 1 liegt.

33. Zusammensetzung nach Anspruch 14 oder einem der Ansprüche 25 bis 32, **dadurch gekennzeichnet, dass** sie 0,1 bis 25 % Füllstoff mit spezifischer Oberfläche, insbesondere 0,5 bis 20 % und besonders 1 bis 15 Gew.-%, bezogen auf das Gesamtgewicht der Zusammensetzung, enthält.

34. Zusammensetzung nach einem der vorhergehenden Ansprüche, **dadurch gekennzeichnet, dass** sie eine wässerige Phase umfasst.

35. Zusammensetzung nach einem der vorhergehenden Ansprüche, **dadurch gekennzeichnet, dass** sie eine wässerige Phase enthält, die aus Wasser oder einem Gemisch von Wasser und einem mit Wasser mischbaren organischen Lösungsmittel besteht.

36. Zusammensetzung nach dem vorhergehenden Anspruch, **dadurch gekennzeichnet, dass** das mit Wasser mischbare organische Lösungsmittel unter den niederen Monoalkoholen mit 1 bis 5 Kohlenstoffatomen, Glycolen mit 2 bis 8 Kohlenstoffatomen, Ketonen mit 3 bis 4 Kohlenstoffatomen und Aldehyden mit 2 bis 4 Kohlenstoffatomen ausgewählt ist.

37. Zusammensetzung nach Anspruch 35 oder 36, **dadurch gekennzeichnet, dass** die wässerige Phase in einer Menge von 1 bis 95 Gew.-%, bezogen auf das Gesamtgewicht der Zusammensetzung, vorzugsweise 3 bis 80 Gew.-% und noch bevorzugter 5 bis 60 Gew.-% enthalten ist.

38. Zusammensetzung nach einem der vorhergehenden Ansprüche, **dadurch gekennzeichnet, dass** sie ein flüchtiges Öl enthält.

39. Zusammensetzung nach dem vorhergehenden Anspruch, **dadurch gekennzeichnet, dass** das flüchtige Öl unter den Kohlenwasserstoffölen, Siliconölen oder deren Gemischen ausgewählt ist.

40. Zusammensetzung nach einem der Ansprüche 38 oder 39, **dadurch gekennzeichnet, dass** das flüchtige Öl in einer Menge von 0,1 bis 98 Gew.-%, bezogen auf das Gesamtgewicht der Zusammensetzung, und vorzugsweise 1 bis 65 Gew.-% enthalten ist.

41. Zusammensetzung nach einem der vorhergehenden Ansprüche, **dadurch gekennzeichnet, dass** sie ein nichtflüchtiges Öl enthält.

42. Zusammensetzung nach dem vorhergehenden Anspruch, **dadurch gekennzeichnet, dass** das nichtflüchtige Öl in einer Menge von 0,1 bis 50 Gew.-%, vorzugsweise 0,1 bis 40 Gew.-%, bezogen auf das Gesamtgewicht der Zusammensetzung, und noch besser 0,1 bis 30 Gew.-% enthalten ist.

43. Zusammensetzung nach einem der vorhergehenden Ansprüche, **dadurch gekennzeichnet, dass** sie ein filmbildendes Polymer enthält.

44. Zusammensetzung nach Anspruch 43, **dadurch gekennzeichnet, dass** das filmbildende Polymer in einem Trockensubstanzgehalt von 0,1 bis 60 Gew.-%, bezogen auf das Gesamtgewicht der Zusammensetzung, vorzugsweise 0,5 bis 40 Gew.-% und noch bevorzugter 1 bis 30 Gew.-% enthalten ist.

45. Zusammensetzung nach einem der vorhergehenden Ansprüche, **dadurch gekennzeichnet, dass** sie ein zusätzliches Wachs enthält.

46. Zusammensetzung nach dem vorhergehenden Anspruch, **dadurch gekennzeichnet, dass** das zusätzliche Wachs in einer Menge von 0,1 bis 50 Gew.-%, bezogen auf das Gesamtgewicht der Zusammensetzung, vorzugsweise 0,5 bis 30 Gew.-% und noch besser 1 bis 20 Gew.-% enthalten ist.

47. Zusammensetzung nach dem vorhergehenden Anspruch, **dadurch gekennzeichnet, dass** sie einen grenzflächenaktiven Stoff enthält.

48. Zusammensetzung nach dem vorhergehenden Anspruch, **dadurch gekennzeichnet, dass** sie einen Zusatzstoff enthält, der unter den Farbmitteln, Antioxidantien, Füllstoffen, pastösen Fettsubstanzen, Konservierungsmitteln, Parfums, Neutralisierungsmitteln, Verdickungsmitteln, Vitaminen, Weichmachern und deren Gemischen ausgewählt ist.

49. Zusammensetzung nach einem der vorhergehenden Ansprüche, **dadurch gekennzeichnet, dass** sie kein UV-Filter enthält.

50. Zusammensetzung nach einem der vorhergehenden Ansprüche, **dadurch gekennzeichnet, dass** sie als Mascara, Produkt für die Augenbrauen, Eyeliner, Lidschatten, Wangenrouge, Make-up, Produkt für die Lippen, Produkt zum Schminken des Körpers (semipermanente Tätowierung) oder Produkt zum Schminken der Haare vorliegt.

51. Zusammensetzung nach einem der vorhergehenden Ansprüche, **dadurch gekennzeichnet, dass** sie als Mascara vorliegt.

52. Zusammensetzung nach einem der vorhergehenden Ansprüche, **dadurch gekennzeichnet, dass** sie als Eyeliner, Lidschatten, Wangenrouge, Make-up, Produkt für die Lippen, Produkt zum Schminken des Körpers (semipermanente Tätowierung) vorliegt.

53. Zusammensetzung nach einem der vorhergehenden Ansprüche, **dadurch gekennzeichnet, dass** es sich um eine Zusammensetzung für die Pflege oder zum Schminken von Keratinfasern handelt.

54. Zusammensetzung nach einem der vorhergehenden Ansprüche, **dadurch gekennzeichnet, dass** es sich um eine Mascara handelt.

55. Nichttherapeutisches kosmetisches Verfahren zum Schminken oder zur Pflege von Keratinsubstanzen, das umfasst, auf die Keratinsubstanzen eine Zusammensetzung nach einem der vorhergehenden Ansprüche aufzutragen.

56. Verwendung einer Zusammensetzung nach einem der Ansprüche 1 bis 54, um auf den Keratinsubstanzen eine homogene und/ oder glatte Schminke zu bilden und/ oder die Keratinsubstanzen schnell zu schminken.

57. Verwendung einer Zusammensetzung nach einem der Ansprüche 14 bis 54, um den Wimpern Fülle zu geben und die Wimpern zu trennen.

58. Verwendung eines Wachses mit einer Klebrigkeit von 0,7 N·s oder darüber und einer Härte von 3,5 MPa oder darunter in einer kosmetischen Zusammensetzung, um Keratinsubstanzen schnell und/oder homogen und/oder glatt zu schminken und/oder eine stabile kosmetische Zusammensetzung herzustellen, wobei das Wachs in einer Menge von mindestens 27 Gew.-%, bezogen auf das Gesamtgewicht der Zusammensetzung, enthalten ist.

59. Verwendung mindestens einer Verbindung, die unter einem Ester von Dextrin und einer oder mehreren Fettsäuren und/oder einem Füllstoff mit einer spezifischen BET-Oberfläche von 100 m²/g oder darüber ausgewählt ist, in einer kosmetischen Zusammensetzung, die mindestens 27 Gew.-% mindestens eines Wachses mit einer Klebrigkeit von 0,7 N·s oder darüber und einer Härte von 3,5 MPa oder darunter, bezogen auf das Gesamtgewicht der Zusammensetzung, enthält, um die Keratinsubstanzen homogen und/oder glatt zu schminken, insbesondere um den Keratinfasern Fülle zu geben und die Keratinfasern zu trennen, und/oder um eine stabile kosmetische Zusammensetzung herzustellen.

60. Verwendung nach einem der Ansprüche 58 oder 59, **dadurch gekennzeichnet, dass** das Wachs ein Alkyl(C₂₀₋₄₀)(hydroxystearoyloxy)stearat der folgenden Formel (I) ist: 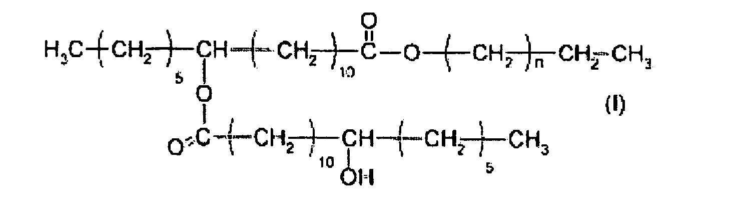 worin n eine ganze Zahl von 18 bis 38 bedeutet, oder ein Gemisch von Verbindungen der Formel (I).

61. Einheit (1) zur Konfektionierung und zum Auftragen eines Produktes zur Umhüllung von Keratinfasern, die umfasst:
i) einen Behälter (2), der eine Zusammensetzung nach einem der Ansprüche 1 bis 53 enthält, und
ii) Einrichtungen (12) insbesondere in Form einer gedrehten Bürste oder eines Kamms zum Aufbringen der Zusammensetzung auf die Fasern.
